# EUROPEAN PATENT SPECIFICATION

(11) **EP 4 046 293 B1**
(45) Date of publication and mention of the grant of the patent: **09.12.2026**
(21) Application number: 20796942.9
(22) Date of filing: 15.10.2020
(51) Int. Cl.: H04B 10/114, A61B 5/1455, H04B 13/00

(54) **OPTICAL TRANSMISSION FOR AN IMPLANTABLE SYSTEM**
OPTISCHE ÜBERTRAGUNG FÜR EIN IMPLANTIERBARES SYSTEM
TRANSMISSION OPTIQUE POUR SYSTÈME IMPLANTABLE

(30) Priority: 16.10.2019 US 201962915967 P
(43) Date of publication of application: 24.08.2022
(73) Proprietor: Ability Neurotech SA, 1202 Geneva (CH)
(72) Inventor: BURDIN, Florent, 1202 Genève (CH); BERTOLOTE, Tiago, 1202 Genève (CH); COQUOZ, Olivier, 1202 Genève (CH)
(74) Representative: Marks & Clerk LLP
(86) International application number: PCT/IB2020/059735
(87) International publication number: WO 2021/074866

(56) References cited:
- US-A1- 2009 163 968
- US-A1- 2012 116 155
- ACKERMANN D M ET AL: "Designing the Optical Interface of a Transcutaneous Optical Telemetry Link", IEEE TRANSACTIONS ON BIOMEDICAL ENGINEERING, IEEE SERVICE CENTER, PISCATAWAY, NJ, USA, vol. 55, no. 4, 1 April 2008 (2008-04-01), pages 1365 - 1373, XP011342675, ISSN: 0018-9294, DOI: 10.1109/TBME.2007.913411

## Description

### RELATED APPLICATION

This application claims the benefit of U.S. Provisional Application No. 62/915,967, filed on October 16, 2019.

### BACKGROUND

Monitoring devices designed for implant in the human body require a way to transmit the data that they collect to an external device so that the collected data can be processed. Typically, such implantable devices employ optical or radio frequency transmission.
US2009163968 discloses an implantable medical device for monitoring tissue perfusion that includes a light source emitting light having a light wavelength corresponding to a blue to ultraviolet light spectrum and a light detector receiving light emitted by the light source and scattered by a volume of body tissue. The light detector emits a signal correlated to the received light wavelength, and a processor receives the signal from the light detector and determines a patient condition in response to the signal.
US2012116155 discloses a surgical device which includes an optical source for wirelessly transmitting a light based signal transcutaneously and a receiver for receiving the light based signals. The wireless coupling of signals between the optical source and the receiver wirelessly transmits video images from an internal site in a patient to a video monitor or other viewer outside the patient, and may wirelessly transmit control signals from a controller outside of the patient to an instrument inside the patient during a therapeutic or diagnostic surgical procedure. US2012116155 discloses an embodiment in which an external unit 20 has a plurality of receivers 26 and a plurality of optical sources 30 for wirelessly transmitting light based signals transcutaneously. Similarly, an internal unit 40 has a plurality of optical sources 50 and a plurality of receivers 46. Each of the plurality of receivers 26/46 is optically configured for receiving the light based signals from a different one of the plurality of optical sources 30/50 to define optically coupled pairs.
"Designing the Optical Interface of a Transcutaneous Optical Telemetry Link" by Ackermann et al describes a mathematical analysis and supporting data that quantitatively describes the relationship between primary interface design parameters of a transmitter-skin-receiver interface. These parameters include the thickness of the skin through which the light is transmitted, the size of the integration area of the optics, the degree of transmitter-receiver misalignment, the efficiency of the optics system, and the emitter power. The particular combination of these parameters chosen for the hardware device will determine the receiver signal power and, therefore, the data quality for the link. This paper demonstrates some of the tradeoffs involved in the selection of these design parameters and provides suggestions for link design.

### SUMMARY

Embodiments of the present disclosure are directed to allowing a safe and robust optical transfer of data through biological tissues with a high throughput and high penetration depth, while being highly tolerant to misalignment and minimizing optical power density and tissue temperature increase.

An example embodiment includes multiple light sources separated by a certain distance within an implanted hermetic housing, with multiple robust low-profile optical windows that allow light to exit the hermetic housing and to be safely injected into the biological tissue. The optical window geometry and/or surface properties may be adjusted to positively influence light propagation through the tissue. One or more photodiodes receive the optical signal on the other side of the tissue and convert the received optical signal into an electric signal. The electrical signal is amplified by one or more amplifiers that feed a clock and data recovery stage.

According to an example embodiment, a transcutaneous optical communication system according to claim 1 includes an implantable optical transmitter device and an external optical receiver device.

The implantable optical transmitter device may include a hermetic housing having a cavity, a distal end, and a proximal end, the cavity including one or more drivers, plural light emitting sources, and an optical element arranged therein. Each of the one or more drivers is configured to convert a digital data signal into one or more modulation signals to drive one or more of the light emitting sources. Each light emitting source is configured to generate a light beam in response to a corresponding one of the one or more modulation signals, each light beam contributing to form a single optical signal. The optical element is configured to direct the light beams to exit the proximal end of the hermetic housing distributed in a pattern in which a peak position of light intensity of each light beam is separated from a corresponding peak position of light intensity of an adjacent light beam by at least a first distance and less than a second distance.

The external optical receiver device may include at least one photodiode configured to detect light generated by the plural light emitting sources and to responsively generate an external detection signal, amplifier circuitry configured to amplify the external detection signal, and clock and data recovery circuitry coupled to receive the amplified detection signal and configured to generate a reconstructed data signal.

According to an example embodiment, a method for transcutaneous optical communication according to claim 10 includes, at an implantable optical transmitter device, converting a digital data signal into one or more modulation signals, generating a light beam in response to a corresponding one of the one or more modulation signals, each light beam contributing to form a single optical signal, and directing the light beams to exit the implantable optical transmitter device distributed in a pattern in which a peak position of light intensity of each light beam is separated from a corresponding peak position of light intensity of an adjacent light beam by at least a first distance and less than a second distance.

The method may further include, at an external optical receiver device positioned to detect one or more of the light beams, detecting light generated by the plural light emitting sources and responsively generating an external detection signal, amplifying the external detection signal, and receiving the amplified detection signal and generating a reconstructed data signal.

According to an example embodiment, an implantable device comprises a hermetic housing having a cavity, a distal end, and a proximal end, the cavity including one or more drivers, plural light emitting sources, and an optical element arranged therein. Each of the one or more drivers is configured to convert a digital data signal (representing a physiological signal) into one or more modulation signals to drive one or more of the light emitting sources. Each light emitting source is configured to generate a light beam in response to a corresponding one of the one or more modulation signals, each light beam contributing to form a single optical signal. The optical element is configured to direct the light beams to exit the proximal end of the hermetic housing distributed in a pattern in which a peak position of light intensity of each light beam is separated from a corresponding peak position of light intensity of an adjacent light beam by at least a first distance and less than a second distance.

The implantable device may be configured to be embedded within biological tissue and the first distance and the second distance are based on characteristics of the biological tissue.

In an example embodiment, the first distance may be greater than 0.5 millimeters and the second distance less than 50 millimeters.

In an example embodiment, the optical element comprises plural optical windows. Each optical window of the plural optical windows may comprise a lens, an anti-reflective coating, a diffusing layer, a micro-structured surface, or any combination thereof.

The implantable device may further include a ferrule positioned at the proximal end of the housing and configured to contain the plural optical windows, the ferrule having plural openings aligned with the plural optical windows, the plural optical windows recessed from a top surface of the ferrule.

The plural light emitting sources may comprise N light emitting sources, and the plural optical windows may comprise M optical windows, with N greater than or equal to M.

In an example embodiment, the optical element comprises a single optical window. The single optical window may comprise a lens, an anti-reflective coating, a diffusing layer, a micro-structured surface, or any combination thereof.

The implantable device may further include a ferrule having plural openings, the ferrule positioned at the proximal end of the housing and the single optical window recessed from the proximal end of the housing by at least a thickness of the ferrule.

The plural light emitting sources may comprise N light emitting sources, and the plural openings may comprise M openings, with N greater than or equal to M.

The one or more drivers may be configured to operate based on on-off keying modulation and/or multiple amplitude shift keying modulation.

The implantable device may further include analog front-end circuitry configured to convert a physiological signal to the digital data signal.

According to an example embodiment, a transcutaneous optical communication system includes an external optical transmitter device and an implantable optical receiver device.

The external optical transmitter device may include a housing having one or more drivers, plural light emitting sources, and an optical element arranged therein. Each of the one or more drivers is configured to convert a digital data signal into one or more modulation signals to drive one or more of the light emitting sources. Each light emitting source is configured to generate a light beam in response to a corresponding one of the one or more modulation signals, each light beam contributing to form a single optical signal. The optical element is configured to direct the light beams to exit the housing distributed in a pattern in which a peak position of light intensity of each light beam is separated from a corresponding peak position of light intensity of an adjacent light beam by at least a first distance and less than a second distance.

The implantable optical receiver device may include at least one photodiode configured to detect light generated by the plural light emitting sources and to responsively generate an external detection signal, amplifier circuitry configured to amplify the external detection signal, a receiver coupled to receive the amplified detection signal and configured to generate a reconstructed data signal, a controller configured to convert the reconstructed data signal to a controller signal, and a stimulation generator configured to generate a stimulation signal based on the controller signal.

### BRIEF DESCRIPTION OF THE DRAWINGS

The foregoing will be apparent from the following more particular description of example embodiments, as illustrated in the accompanying drawings in which like reference characters refer to the same parts throughout the different views. The drawings are not necessarily to scale, emphasis instead being placed upon illustrating embodiments.
FIG. 1 shows a conceptual view of a transcutaneous optical communication system.
FIG. 2 illustrates a block diagram of a first embodiment of a transcutaneous optical communication system.
FIGs. 3A-3B illustrate an optical beam shape after going through optical phantoms in an experimental configuration.
FIGs. 4A-4D show several example arrangements for an optical element.
FIGs. 5A-5B show example arrangements of an optical element in which four emitting sources are employed.
FIGs. 6 and 7 show example arrangements of an impactor in relation to example optical windows.
FIGs. 8A-8B illustrate in more detail an example multiple window and ferrule arrangement.
FIG. 9 illustrates a block diagram of a second embodiment of a transcutaneous optical communication system.

### DETAILED DESCRIPTION

A description of example embodiments follows.

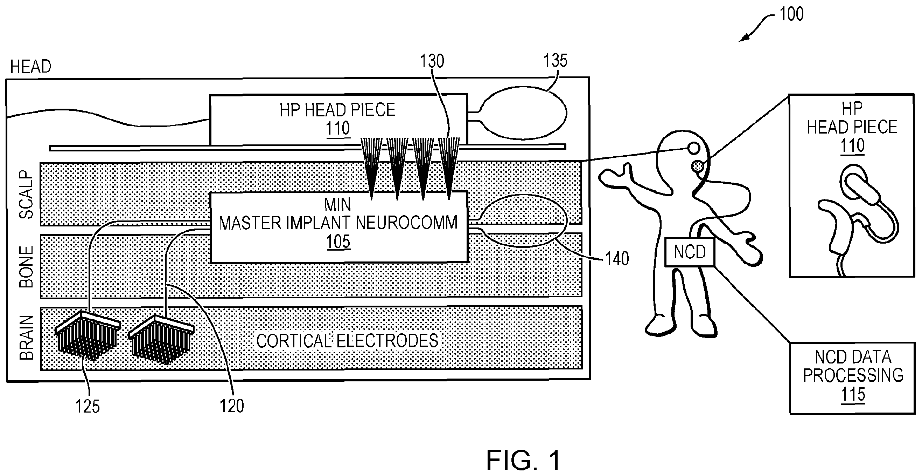

FIG. 1 illustrates a conceptual view of a transcutaneous communication system 100. The system 100 includes a master implant neuro-communicator (MIN) 105, an external head piece (HP) or wearable device 110, and a data processing device (NCD) 115. As shown conceptually, the MIN 105 is implanted under a patient's scalp and has signal wires 120 coupled to cortical electrodes 125 in contact with a portion of the patient's brain cortex. The MIN 105 converts physiological signals received on the signal wires 120 to an optical signal 130 that is transmitted to the HP 110 through the scalp. The HP 110 receives the optical signal on the other side of the scalp and converts the received optical signal into an electric signal. The electrical signal is amplified by one or more amplifiers, which feed a clock and data recovery stage to produce a reconstructed data signal. The NCD 115 is configured for data processing functions on the reconstructed data signal. The HP 110 is configured to power and control the MIN 105 via coils 135, 140.

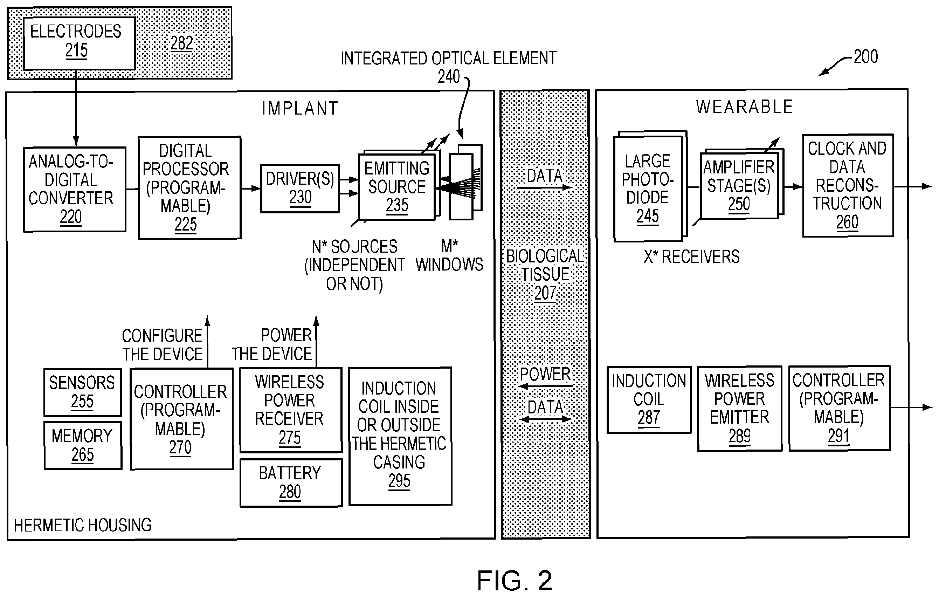

FIG. 2 illustrates a block diagram of a first example embodiment of a transcutaneous communication system 200. The system 200 includes an implantable device or a master implant neuro-communicator (MIN) 205 and an external wearable device 210. The MIN 205 has a hermetic housing (not shown) that includes an analog-to-digital converter (ADC) 220, a programmable digital signal processor (DSP) 225, one or more drivers 230, plural light emitting sources 235, and an integrated optical element 240 that includes plural windows. Electrodes 215 provide analog physiological signals to the ADC 220 when attached to a patient's cortical region 282. It should be understood that other regions of the body can also be contemplated for use with embodiments configured based on functions and elements of the MIN 205.

The MIN 205 may further include sensors 255, memory 265, a controller 270 for configuring the MIN 205, a wireless power receiver 275 for powering the MIN 205, battery 280, and an induction coil 295.

The sensors 255 may include temperature sensors, humidity sensors, voltage and current sensors, accelerometers, etc. The sensors 255 are useful for monitoring the MIN 205 and to ensure safety.

The memory 265 may be arranged to store the implant's configurations, firmware, implant and/or patient information (e.g., name, serial number) and/or to log data (e.g., battery voltage, temperature, humidity, time, events).

The controller 270 may be a programmable microcontroller arranged to configure acquisition, the DSP 225, and the drivers. In addition, the controller 270 may be configured to read the sensors 255, to write/read the memory 265, to manage the communication with the wearable 210, and to upgrade the implant's firmware.

The wireless power receiver 275 is configured to convert AC voltage from induction coil 295 into rectified and regulated clean voltages (DC) to power the implant's electronics.

The battery 280 in some embodiments can store energy to be used during power interruption, or to power the MIN 205 when the wearable 210 is absent to keep some functions running.

The induction coil 295 is configured to convert an alternating magnetic field into an alternating electrical signal.

The wearable device 210 includes one or more photodiodes 245, one or more amplifier stages 250, and clock and data recovery circuitry 260.

The wearable device 210 further includes an induction coil 287, a wireless power emitter 289, and a controller 291.

In operation, the ADC 220 converts the analog physiological signal received from electrodes 215 to a digital signal. The ADC function may be provided by, for example, an analog front end (AFE) chip. The DSP 225 processes the digital signal. The DSP 225 controls the ADC 220, reads the result of the analog-to-digital conversions, encapsulates the data with a header and a checksum to ensure data integrity and sends the data to one or more drivers 230. The output of the DSP 225 is coupled to the one or more drivers 230 which convert the digital signal to one more modulation signals to drive the plural light emitting sources 235. The light beams emitted from the emitting sources 235 contribute to form a single optical signal. The integrated optical element 240 has multiple robust low profile optical windows that allow the optical signal to exit the hermetic housing and to be safely injected into the biological tissue 207. At the wearable device 210, one or more photodiodes 245 receive the optical signal on the other side of the tissue and convert the received optical signal into an electric signal. In alternative embodiments, other types of optical receivers are used in place of the photodiodes 245. The electrical signal is amplified by one or more amplifiers 250, which feed a clock and data recovery or reconstruction stage 260. The reconstructed data signal exits 262 the wearable device 210 for further processing.

The wearable device 210 may be configured to transfer power from wireless power emitter 289 to the wireless power receiver 275 at MIN 205 via the induction coils 287, 295. In addition, the wearable device 210 may be configured to program and communicate with the controller 270 at MIN 205 from controller 291 via the induction coils 287, 295.

### Target Data Rate

New applications of implantable devices require large amounts of information to transit across the patient's tissue, which can typically occur at a date transfer rate of more than 25 Mbps, given the number of channels, sampling rate, and resolution required.

### Skin Thickness

The optical power that needs to be transmitted through tissue is affected by tissue thickness and type. In an example of potential application of device implanted in a patient's head, the tissue thickness can be typically 7 to 8 mm in average, reaching up to 12 mm, or even more.

### Wavelengths

The skin absorption and scattering coefficients are not constant and vary with the wavelength. The ability of light to penetrate biological tissues depends also on tissue components such as pigments, melanin, fat, water, and oxy/deoxy blood. Therefore, the wavelength is chosen to be able to maximize the transmitted energy, but the link is also configured to be flexible and tolerant enough to accommodate all skin variations.

Many publications identified a "near-infrared window" in biological tissues, between 600nm and 1300nm. At these wavelengths, the combination of the melanin, the water, and the blood absorbes less light than at shorter or longer wavelengths. Plus, the scattering coefficient of biological tissues decreases when the wavelength increases. In some embodiments, the wavelength may be in the range between 400nm and 1400nm.

### Emitting Source

The optical communication link, unidirectional, is based on multiple fast emitting infrared sources, for power adjustment and redundancy (and to decrease the power density by spreading the power over the multiple sources), modulated by the digital data with the operation of a driver. The scalp thickness, absorption, and scattering properties can be compensated by an adjustable emitting source and an adjustable receiver sensitivity. Therefore, it is possible to optimize the bit error rate while keeping the power consumption as low as possible.

The emitting sources 235 can be light emitting diodes (LEDs) or vertical-cavity surface-emitting lasers (VCSELs). Both are types of emitting sources that can emit infrared radiations in a small form factor. While LEDs are typically limited to 20Mbps, VCSELs can achieve a data rate up to several Gbps.

### Driver

In an embodiment, the driver(s) 230 may be a very simple and high-speed transistor used to modulate the VCSEL current with a power efficient and simple On Off Keying (OOK) modulation. The multiple emitting sources can also be operated using multiple amplitude shift keying (M-ASK).

### Wearable photodiode

The photodiode 245 may be, for example, a Hamamatsu S6967 photodiode, which has a 50MHz bandwidth, a sensitivity of about 0.62 W/A at 850nm, a photosensitive area of 26.4 mm², and a large viewing angle of more than 120°. Placing the photodiode 245 as close as possible to the skin has the advantage of providing a large viewing angle, which also aids with alignment to the emitting sources. The goal is to be able to capture all the diffused light reaching the surface of the skin, even if the photons arrive with a significant incident angle. An optical system (e.g., Fresnel lens, lens, filter) (not shown) may be added to the photodiode to capture more photons or to select only the wavelength of interest.

### Amplifier stages

The photodiode 245 delivers a current proportional to the optical power received. The amplifier stage(s) 250 transform this current into a voltage.

In an experimental configuration to demonstrate the concepts of the implantable devices described herein, solid optical phantoms have been used to mimic the optical properties of the tissue. Nominal and extreme cases for optical properties and tissue thickness (2mm and 15mm representing the extremes for skin thickness) have been used.

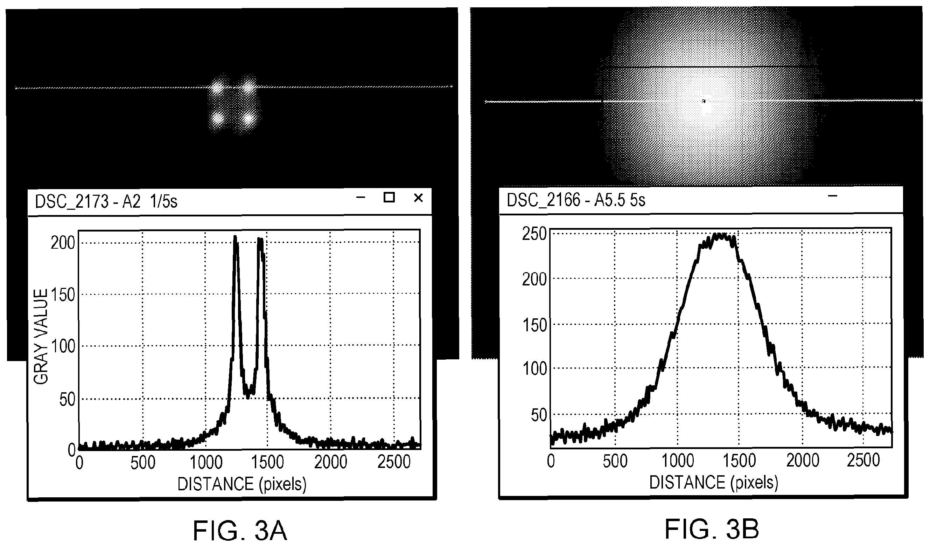

FIGs. 3A-3B illustrate the shape of the beam after going through optical phantoms in the experimental configuration. FIG. 3A shows results for an optical phantom A2, which is 2mm thick, and FIG. 3B shows results for an optical phantom A5.5, which is 5.5mm thick. For the A2 phantom, distinct optical beams from four emitter sources can be seen separated. For phantoms thicker than 2mm, such as the A5.5 phantom, the beams are intersecting and summed to form a single peak, as shown by FIG. 3B, thanks to the scattering effect. Therefore, for thin skin the multiple sources concept increases the alignment tolerance, and for thick skins the total beam formed is a combination of the four beams, and the alignment tolerance is probably larger due to the scattering coefficient.

It has been found that, in order to accommodate ranges of thickness of the biological tissue 207 (FIG. 2), the emitting sources are separated such that the light beams that exit the hermetic housing are distributed in a pattern in which a peak position of light intensity of each light beam is separated from a corresponding peak position of light intensity of an adjacent light beam by at least a first distance and less than a second distance. For example, the first distance may be greater than 0.5 mm, and the second distance may be less than 50 mm.

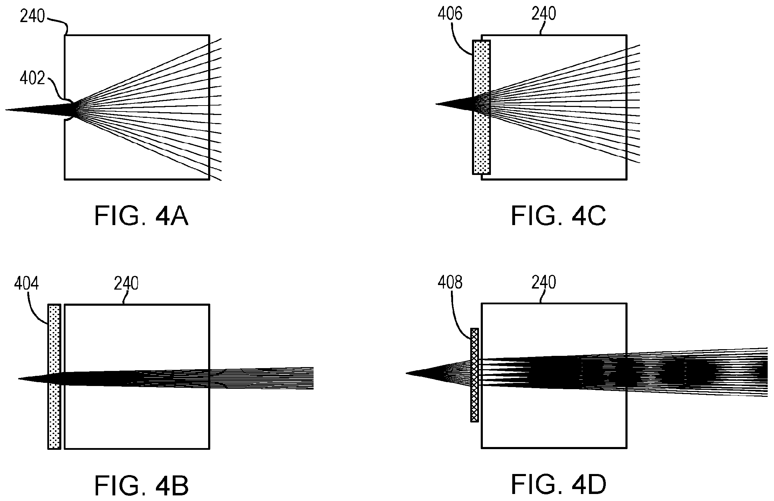

The integrated optical element 240 can be, for example, constructed from sapphire or other suitable material. FIGs. 4A-4D show several example arrangements for the optical element 240. In FIG. 4A the optical element includes a lens 402 such as a plano-concave lens. In FIG. 4B an anti-reflective coating 404 (e.g., thin film or thick film) is applied to a surface of the optical element 240 to reduce reflection and therefore increase the transmitted energy. In FIG. 4C a diffusing layer 406, such as a frosted surface, is applied to a surface of the optical element 240. In FIG. 4D the optical element 240 includes a micro-structured or micro-patterned surface 408.

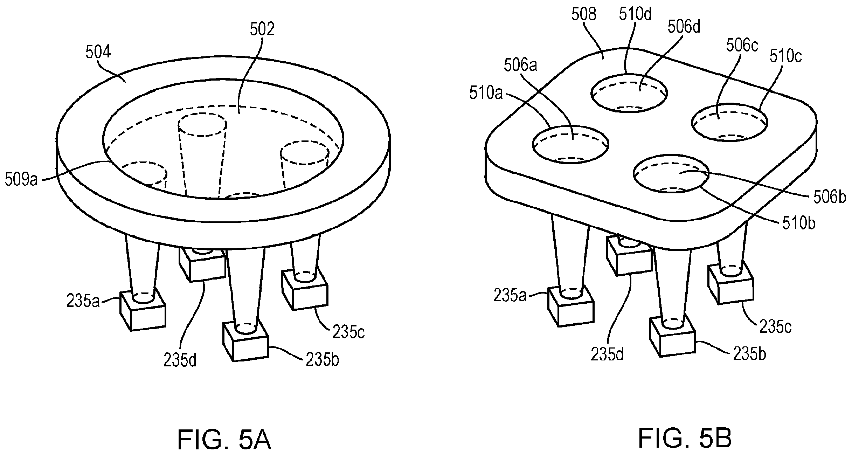

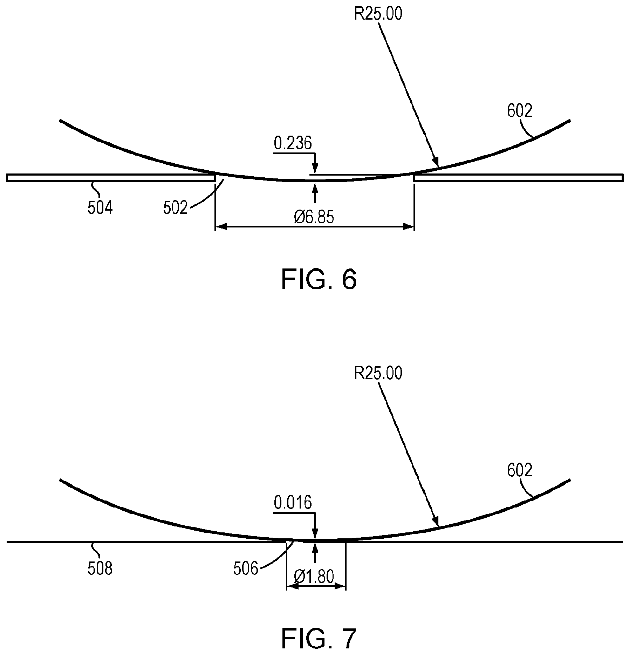

FIGs. 5A-5B show two example arrangements of the optical element 240 in which four emitting sources 235a-235d are employed. In FIG. 5A the optical element comprises a single window 502 held in place by a ferrule 504 having a single opening 509a. In FIG. 5B the optical element comprises four windows 506a-506d held in place by a ferrule 508 having four corresponding openings 510a-510d. Each one of the multiple optical windows 506a-506d, covering a subset of infrared emitting sources 235a-235d, can have a smaller diameter than a single window covering all sources. For a given thickness, multiple windows have a lower diameter-to-thickness ratio than a single larger window, therefore being more mechanically robust. Similarly, with a given recess distance from the surface of the windows to the surface of their supporting ferrule, an impactor having a given concave surface can directly hit the surface of the larger single window, while avoiding the surface of the smaller ones. Put another way, for a given impactor with a given concave surface, the surface of the larger single window needs to be recessed further than the surface of the smaller windows to avoid an impact. This concept is shown in FIGs. 6 and 7, which show an impactor 602 having radius 25mm. In FIG. 6, the large window 502 having a 6.85mm diameter is recessed by 0.236mm. By contrast, in FIG. 7 the small window 506 having a 1.80mm diameter only needs to be recessed by 0.016mm to avoid the impactor 602. An advantage of the smaller window 506 is that the packaging of the implantable device can be made smaller since the recess is smaller.

The combination of a direct impact and a high diameter-to-thickness ratio generates an increased probability of damage to the window, possibly resulting in a loss of hermeticity: recess distance and/or window thickness would need to be increased to avoid that, and both these options would hinder the "low profile" aspect.

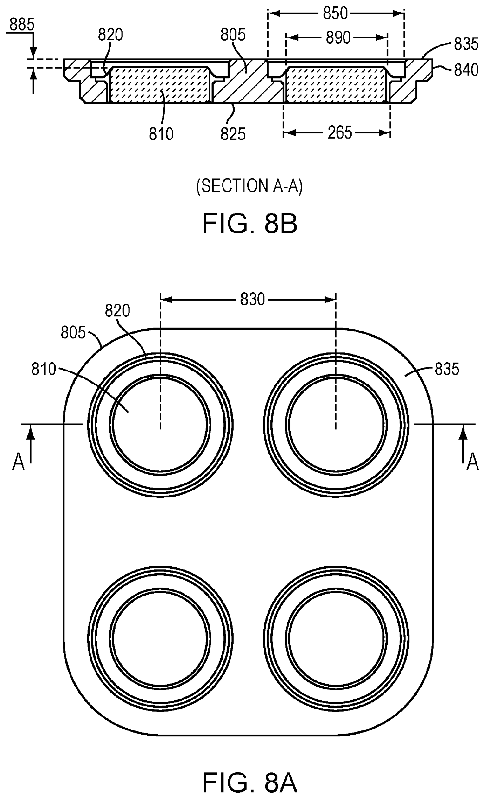

FIGs. 8A-8B illustrate in more detail an example multiple window and ferrule arrangement. FIG. 8A is a plan view that shows ferrule 805 holding four sapphire windows 810. The ferrule 805 may be made from titanium or other suitable material. The centers of the windows 810 are separated by a distance 830. As shown in the cross-sectional view (FIG. 8B) through a cutting plane A-A illustrated by lines A-A, the windows 810 are recessed from the top 835 of the ferrule by a distance 885. In this embodiment, the windows 810 are not recessed from the bottom 825 of the ferrule, but in other embodiments, the windows may be recessed from bottom also. The ferrule is bonded to each window 810 by a hermetic seal 820, by means of a pure gold brazing for example, and includes a flange 840 for seating the ferrule in the housing. The distance 830 between centers of windows 810 is selected to correspond with placement of the one or more emitting sources 235 (FIG. 2) for alignment with the windows 810.

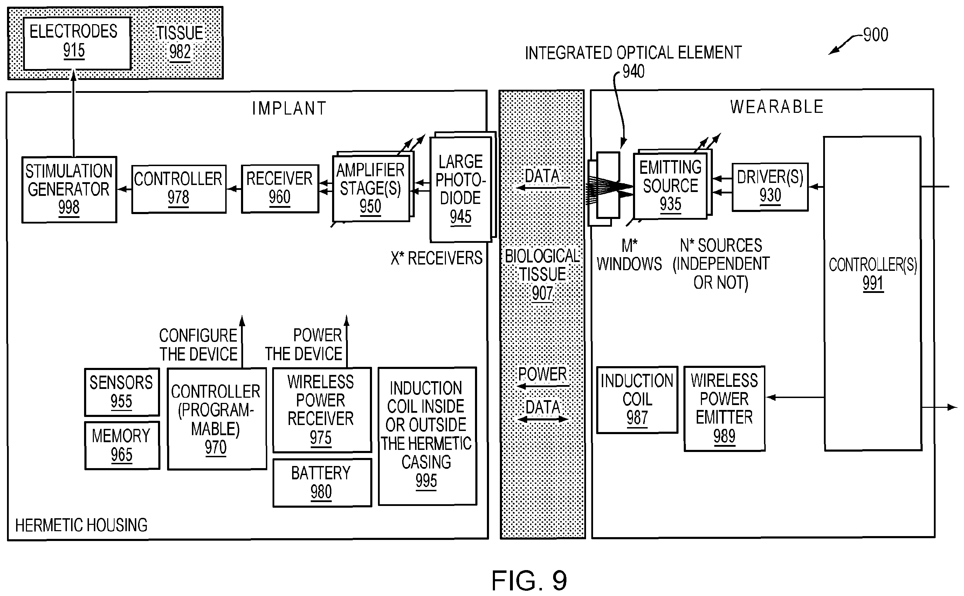

FIG. 9 illustrates a block diagram of a second example embodiment of a transcutaneous optical communication system. In this system 900, optical communication is directed from a wearable device 910 to an implant device 905. Such a system 900 may be configured to deliver stimulation signals internally to an area of tissue. In addition, the system 900 may be configured to provide service signals for programming, upgrading and/or changing parameters of the implant device 905.

The implant device 905 has a hermetic housing (not shown) that includes one or more photodiodes 945, one or more amplifier stages 950, receiver 960, controller 978, and stimulation generator 998. Electrodes 915 provide analog physiological signals from stimulation generator 998 when attached to a tissue region of a patient.

The implant device 905 may further include sensors 955, memory 965, a controller 970 for configuring the implant device 905, a wireless power receiver 975 for powering the implant device 905, battery 980, and an induction coil 995.

The sensors 955 may include temperature sensors, humidity sensors, voltage and current sensors, accelerometers, etc. The sensors 955 are useful for monitoring the implant device 905 and to ensure safety.

The memory 965 may be arranged to store the implant's configurations, firmware, implant and/or patient information (e.g., name, serial number) and/or to log data (e.g., battery voltage, temperature, humidity, time, events).

The controller 970 may be a microcontroller arranged to configure controller 978. In addition, the controller 970 may be configured to read the sensors 955, to write/read the memory 965, to manage the communication with the wearable 910 and to upgrade the implant's firmware.

The wireless power receiver 975 is configured to convert AC voltage from induction coil 995 into rectified and regulated clean voltages (DC) to power the implant's electronics.

The battery 980 in some embodiments can store energy to be used during power interruption, or to power the implant device 905 when the wearable 910 is absent to keep some functions running.

The induction coil 995 is configured to convert an alternating magnetic field into an alternating electrical signal.

The wearable device 910 includes one or more drivers 930, plural light emitting sources 935, and an integrated optical element 940 that includes plural windows.

The wearable device 910 further includes an induction coil 987, a wireless power emitter 989, and a controller 991.

In operation, the controller 991 provides a digital signal to the one or more drivers 930 which convert the digital signal to one more modulation signals to drive the plural light emitting sources 935. The light beams emitted from the emitting sources 935 contribute to form a single optical signal. The integrated optical element 940 has multiple robust low profile optical windows that allow the optical signal to exit the wearable device 910 and to be safely injected into the biological tissue 907. At the implant device 905, one or more photodiodes 945 receive the optical signal on the other side of the tissue and convert the received optical signal into an electric signal. The electrical signal is amplified by one or more amplifiers 950 that feed a receiver 960. The reconstructed data signal is coupled to controller 978, which is programmed to control the stimulation generator 998 to provide stimulation signals to tissue 982.

The wearable device 910 may be configured to transfer power from wireless power emitter 989 to the wireless power receiver 975 at implant device 905 via the induction coils 987, 995. In addition, the wearable device 910 may be configured to program the controller 970 from controller 991 via the induction coils 987, 995.

While example embodiments have been particularly shown and described, it will be understood by those skilled in the art that various changes in form and details may be made therein without departing from the scope of the embodiments encompassed by the appended claims.

## Claims

1. A transcutaneous optical communication system (100, 200) comprising:
an implantable optical transmitter device including:
a hermetic housing having a cavity, a distal end, and a proximal end, the cavity including one or more drivers (230), plural light emitting sources (235), and an optical element (240) arranged therein;
each of the one or more drivers (230) configured to convert a digital data signal into one or more modulation signals to drive one or more of the light emitting sources (235);
each light emitting source (235) configured to generate a light beam (130) in response to a corresponding one of the one or more modulation signals, each light beam (130) contributing to form a combined optical signal; and
the optical element (240) configured to direct the combined optical signal to exit the proximal end of the hermetic housing distributed in a pattern in which a peak position of light intensity of each light beam of the combined optical signal is separated from a corresponding peak position of light intensity of an adjacent light beam of the combined optical signal by at least a first distance and less than a second distance, the first distance being less than the second distance, wherein the implantable optical transmitter device is configured to be embedded within biological tissue (207) and the first distance and the second distance are based on characteristics of the biological tissue (207);
an external optical receiver device (110) including:
at least one photodiode (245) configured to detect the combined optical signal and to responsively generate an external detection signal;
amplifier circuitry (250) configured to amplify the external detection signal; and
clock and data recovery circuitry (260) coupled to receive the amplified detection signal and configured to generate a reconstructed data signal.

2. The system of claim 1 wherein the first distance is greater than 0.5 millimeters and the second distance is less than 50 millimeters.

3. The system of claim 1 wherein the optical element (240) comprises plural optical windows (506a-506d).

4. The system of claim 3, wherein:
each optical window of the plural optical windows (506a-506d) comprises a lens (402), an anti-reflective coating (404), a diffusing layer (406), a micro-structured surface (408) or any combination thereof, preferably wherein the lens is a plano-concave lens (402), or
the implantable optical transmitter device further comprises a ferrule (508, 805) positioned at the proximal end of the housing and configured to contain the plural optical windows (506a-506d), the ferrule having plural openings (510a-510d) aligned with the plural optical windows, the plural optical windows recessed from a top surface of the ferrule, or
the plural light emitting sources (235) comprise N light emitting sources and the plural optical windows comprise M optical windows, with N greater than or equal to M.

5. The system of claim 1 wherein the optical element comprises a single optical window (502), optionally
wherein the single optical window (502) comprises a lens (402), an anti-reflective coating (404), a diffusing layer (406), a micro-structured surface (408) or any combination thereof, or
optionally wherein the implantable optical transmitter device further comprises a ferrule (508, 805) having plural openings (510a-510d), the ferrule positioned at the proximal end of the housing and the single optical window (502) recessed from the proximal end of the housing by at least a thickness of the ferrule,
preferably wherein the plural light emitting sources (235) comprise N light emitting sources and the plural openings comprise M openings, with N greater than or equal to M.

6. The system of claim 1 wherein each of the plural light emitting sources (235) comprises any of a light emitting diode and a vertical-cavity surface-emitting laser.

7. The system of claim 1 wherein a first group of the plural light emitting sources (235) comprises vertical-cavity surface-emitting lasers and a second group of the plural light emitting sources (235) comprises light emitting diodes.

8. The system of claim 1 wherein the plural light emitting sources (235) are configured to operate between 400nm and 1400nm, or between 600nm and 1300nm.

9. The system of claim 1 wherein the one or more drivers (230) are configured to operate based on on-off keying modulation, or
wherein the one or more drivers (230) are configured to operate based on multiple amplitude shift keying modulation, or
wherein the implantable optical transmitter device further comprises analog front-end circuitry configured to convert a physiological signal to the digital data signal.

10. A method for transcutaneous optical communication, the method comprising:
at an implantable optical transmitter device:
converting a digital data signal into one or more modulation signals;
at one or more of plural light emitting sources (235), generating a light beam (130) in response to a corresponding one of the one or more modulation signals, each light beam (130) contributing to form a combined optical signal; and
directing the combined optical signal to exit the implantable optical transmitter device distributed in a pattern in which a peak position of light intensity of each light beam of the combined optical signal is separated from a corresponding peak position of light intensity of an adjacent light beam of the combined optical signal by at least a first distance and less than a second distance, the first distance being less than the second distance, wherein the implantable optical transmitter device is configured to be embedded within biological tissue (207) and the first distance and the second distance are based on characteristics of the biological tissue (207).

11. The method of claim 10 further comprising:
at an external optical receiver device (110) positioned to detect one or more of the light beams (130):
at one or more of plural photodiodes (245), detecting light generated by the plural light emitting sources (235) and responsively generating an external detection signal;
amplifying the external detection signal; and
receiving the amplified detection signal and generating a reconstructed data signal.

12. The method of claim 10 wherein the first distance is greater than 0.5 millimeters and the second distance is less than 50 millimeters.

13. The method of claim 10 wherein the directing is by an optical element comprising plural optical windows (506a-506d).

14. The method of claim 10 wherein the directing is by an optical element comprising a single optical window (502).

15. The method of claim 10 wherein each of the plural light emitting sources (235) comprises any of a light emitting diode and a vertical-cavity surface-emitting laser, or
wherein a first group of the plural light emitting sources (235) comprises vertical-cavity surface-emitting lasers and a second group of the plural light emitting sources (235) comprises light emitting diodes, or
wherein the generating a light beam (130) in response to a corresponding one of the one or more modulation signals is based on on-off keying modulation, or
wherein the generating a light beam (130) in response to a corresponding one of the one or more modulation signals is based on multiple amplitude shift keying modulation, or further comprising converting a physiological signal to the digital data signal.

## Patentansprüche

1. Transkutanes optisches Kommunikationssystem (100, 200), umfassend:
eine implantierbare optische Sendevorrichtung, beinhaltend:
ein hermetisches Gehäuse, das einen Hohlraum, ein distales Ende und ein proximales Ende aufweist, wobei der Hohlraum einen oder mehrere Treiber (230), mehrere Leuchtquellen (235) und ein optisches Element (240) darin angeordnet beinhaltet;
jeden des einen oder der mehreren Treiber (230), die so konfiguriert sind, ein digitales Datensignal in ein oder mehrere Modulationssignale umzuwandeln, um eine oder mehrere der Leuchtquellen (235) anzutreiben;
jede Leuchtquelle (235) so konfiguriert, dass sie in Reaktion auf ein oder mehrere entsprechende Modulationssignale einen Lichtstrahl (130) erzeugt, wobei jeder Lichtstrahl (130) zur Bildung eines kombinierten optischen Signals beiträgt; und
das optische Element (240) so konfiguriert, dass es das kombinierte optische Signal so lenkt, dass es das proximale Ende des hermetischen Gehäuses in einem Muster verteilt verlässt, in dem eine Spitzenposition der Lichtintensität jedes Lichtstrahls des kombinierten optischen Signals von einer entsprechenden Spitzenposition der Lichtintensität eines benachbarten Lichtstrahls des kombinierten optischen Signals um mindestens einen ersten Abstand und weniger als einen zweiten Abstand getrennt ist, wobei der erste Abstand kleiner als der zweite Abstand ist, wobei die implantierbare optische Sendevorrichtung dazu konfiguriert ist, in biologisches Gewebe (207) eingebettet zu werden, und der erste Abstand und der zweite Abstand auf Eigenschaften des biologischen Gewebes (207) basieren;
eine externe optische Empfängervorrichtung (110), beinhaltend:
mindestens eine Fotodiode (245), die dazu konfiguriert ist, das kombinierte optische Signal zu erfassen und daraufhin ein externes Erfassungssignal zu erzeugen;
Verstärkerschaltkreise (250), die dazu konfiguriert sind, das externe Erfassungssignal zu verstärken; und
Takt- und Datenwiederherstellungsschaltkreise (260), die dazu gekoppelt sind, das verstärkte Detektionssignal zu empfangen, und dazu konfiguriert sind, ein rekonstruiertes Datensignal zu erzeugen.

2. System nach Anspruch 1, wobei der erste Abstand größer als 0,5 Millimeter ist und der zweite Abstand kleiner als 50 Millimeter ist.

3. System nach Anspruch 1, wobei das optische Element (240) mehrere optische Fenster (506a-506d) umfasst.

4. System nach Anspruch 3, wobei:
jedes optische Fenster der mehreren optischen Fenster (506a-506d) eine Linse (402), eine Antireflexbeschichtung (404), eine Diffusionsschicht (406), eine mikrostrukturierte Oberfläche (408) oder eine beliebige Kombination davon umfasst, wobei die Linse vorzugsweise eine plankonkave Linse (402) ist, oder
die implantierbare optische Sendevorrichtung ferner eine Presshülse (508, 805) umfasst, die am proximalen Ende des Gehäuses positioniert ist und dazu konfiguriert ist, die mehreren optischen Fenster (506a-506d) zu enthalten, wobei die Presshülse mehrere Öffnungen (510a-510d) aufweist, die mit den mehreren optischen Fenstern ausgerichtet sind, wobei die mehreren optischen Fenster von einer Deckfläche der Presshülse zurückgesetzt sind, oder
die mehreren Leuchtquellen (235) N Leuchtquellen umfassen und die mehreren optischen Fenster M optische Fenster umfassen, wobei N größer oder gleich M ist.

5. System nach Anspruch 1, wobei das optische Element ein einzelnes optisches Fenster (502) umfasst, optional
wobei das einzelne optische Fenster (502) eine Linse (402), eine Antireflexbeschichtung (404), eine Diffusionsschicht (406), eine mikrostrukturierte Oberfläche (408) oder eine beliebige Kombination davon umfasst, oder
optional wobei die implantierbare optische Sendevorrichtung ferner eine Presshülse (508, 805) umfasst, die mehrere Öffnungen (510a-510d) aufweist, wobei die Presshülse am proximalen Ende des Gehäuses positioniert ist und das einzelne optische Fenster (502) vom proximalen Ende des Gehäuses um mindestens eine Dicke der Presshülse zurückgesetzt ist,
wobei die mehreren Leuchtquellen (235) vorzugsweise N Leuchtquellen umfassen und die mehreren Öffnungen M Öffnungen umfassen, wobei N größer oder gleich M ist.

6. System nach Anspruch 1, wobei jede der mehreren Leuchtquellen (235) ein beliebiges von einer Leuchtdiode und einem oberflächenemittierenden Laser mit vertikalem Resonator umfasst.

7. System nach Anspruch 1, wobei eine erste Gruppe der mehreren Leuchtquellen (235) oberflächenemittierende Laser mit vertikalem Resonator umfasst und eine zweite Gruppe der mehreren Leuchtquellen (235) Leuchtdioden umfasst.

8. System nach Anspruch 1, wobei die mehreren Leuchtquellen (235) dazu konfiguriert sind, zwischen 400 nm und 1400 nm oder zwischen 600 nm und 1300 nm zu arbeiten.

9. System nach Anspruch 1, wobei der eine oder die mehreren Treiber (230) dazu konfiguriert sind, basierend auf Ein-Aus-Tastungsmodulation zu arbeiten, oder
wobei der eine oder die mehreren Treiber (230) dazu konfiguriert sind, basierend auf Mehrfachamplitudenumtastungsmodulation zu arbeiten, oder
wobei die implantierbare optische Sendevorrichtung ferner analoge Eingangsschaltkreise umfasst, die dazu konfiguriert sind, ein physiologisches Signal in das digitale Datensignal umzuwandeln.

10. Verfahren zur transkutanen optischen Kommunikation, wobei das Verfahren umfasst:
an einer implantierbaren optischen Sendevorrichtung:
Umwandeln eines digitalen Datensignals in ein oder mehrere Modulationssignale;
an einer oder mehreren mehrerer Leuchtquellen (235), Erzeugen, in Reaktion auf ein oder mehrere entsprechende Modulationssignale, eines Lichtstrahls (130), wobei jeder Lichtstrahl (130) zur Bildung eines kombinierten optischen Signals beiträgt; und
Lenken des kombinierten optischen Signals, sodass es die implantierbare optische Sendevorrichtung in einem Muster verteilt verlässt, in dem eine Spitzenposition der Lichtintensität jedes Lichtstrahls des kombinierten optischen Signals von einer entsprechenden Spitzenposition der Lichtintensität eines benachbarten Lichtstrahls des kombinierten optischen Signals um mindestens einen ersten Abstand und weniger als einen zweiten Abstand getrennt ist, wobei der erste Abstand kleiner als der zweite Abstand ist, wobei die implantierbare optische Sendevorrichtung dazu konfiguriert ist, in biologisches Gewebe (207) eingebettet zu werden, und der erste Abstand und der zweite Abstand auf Eigenschaften des biologischen Gewebes (207) basieren.

11. Verfahren nach Anspruch 10, ferner umfassend:
an einer externen optischen Empfängervorrichtung (110), die dazu positioniert ist, einen oder mehrere der Lichtstrahlen (130) zu erfassen:
an einer oder mehreren mehrerer Fotodioden (245), Erfassen von Licht, das von den mehreren Leuchtquellen (235) erzeugt wird, und daraufhin Erzeugen eines externen Erfassungssignals;
Verstärken des externen Erfassungssignals; und
Empfangen des verstärkten Erfassungssignals und Erzeugen eines rekonstruierten Datensignals.

12. Verfahren nach Anspruch 10, wobei der erste Abstand größer als 0,5 Millimeter ist und der zweite Abstand kleiner als 50 Millimeter ist.

13. Verfahren nach Anspruch 10, wobei das Lenken durch ein optisches Element erfolgt, das mehrere optische Fenster (506a-506d) umfasst.

14. Verfahren nach Anspruch 10, wobei das Lenken durch ein optisches Element erfolgt, das ein einzelnes optisches Fenster (506a-506d) umfasst.

15. Verfahren nach Anspruch 10, wobei jede der mehreren Leuchtquellen (235) ein beliebiges von einer Leuchtdiode und einem oberflächenemittierenden Laser mit vertikalem Resonator umfasst, oder
wobei eine erste Gruppe der mehreren Leuchtquellen (235) oberflächenemittierende Laser mit vertikalem Resonator umfasst und eine zweite Gruppe der mehreren Leuchtquellen (235) Leuchtdioden umfasst, oder
wobei das Erzeugen eines Lichtstrahls (130) in Reaktion auf ein entsprechendes des einen oder der mehreren Modulationssignale auf Ein-Aus-Tastungsmodulation basiert, oder
wobei das Erzeugen eines Lichtstrahls (130) in Reaktion auf ein entsprechendes des einen oder der mehreren Modulationssignale auf Mehrfachamplitudenumtastungsmodulation basiert, oder ferner Umwandeln eines physiologischen Signals in das digitale Datensignal umfasst.

## Revendications

1. Système de communication optique transcutanée (100, 200) comprenant :
un dispositif émetteur optique implantable incluant :
un boîtier hermétique présentant une cavité, une extrémité distale et une extrémité proximale, la cavité incluant un ou plusieurs pilotes (230), plusieurs sources d'émission de lumière (235) et un élément optique (240) agencés dans celui-ci ;
chacun des un ou plusieurs pilotes (230) étant configuré pour convertir un signal de données numériques en un ou plusieurs signaux de modulation pour piloter une ou plusieurs des sources d'émission de lumière (235) ;
chaque source d'émission de lumière (235) étant configurée pour générer un faisceau lumineux (130) en réponse à un signal de modulation correspondant des un ou plusieurs signaux de modulation, chaque faisceau lumineux (130) contribuant à former un signal optique combiné ; et
l'élément optique (240) étant configuré pour orienter le signal optique combiné afin qu'il sorte de l'extrémité proximale du boîtier hermétique, distribué selon un modèle dans lequel une position de pic d'intensité de lumière de chaque faisceau lumineux du signal optique combiné est séparée d'une position de pic correspondante d'intensité de lumière d'un faisceau lumineux adjacent du signal optique combiné par au moins une première distance et moins d'une seconde distance, la première distance étant inférieure à la seconde distance, dans lequel le dispositif émetteur optique implantable est configuré pour être intégré dans le tissu biologique (207) et la première distance et la seconde distance sont basées sur des caractéristiques du tissu biologique (207) ;
un dispositif récepteur optique externe (110) incluant :
au moins une photodiode (245) configurée pour détecter le signal optique combiné et pour générer en réponse un signal de détection externe ;
une circuiterie d'amplification (250) configurée pour amplifier le signal de détection externe ; et
une circuiterie d'horloge et d'extraction de données (260) couplée pour recevoir le signal de détection amplifié et configurée pour générer un signal de données reconstruit.

2. Système selon la revendication 1, dans lequel la première distance est supérieure à 0,5 millimètre et la seconde distance est inférieure à 50 millimètres.

3. Système selon la revendication 1, dans lequel l'élément optique (240) comprend plusieurs fenêtres optiques (506a-506d).

4. Système selon la revendication 3, dans lequel :
chaque fenêtre optique des multiples fenêtres optiques (506a-506d) comprend une lentille (402), un revêtement antireflet (404), une couche de diffusion (406), une surface microstructurée (408) ou toute combinaison de ceux-ci, de préférence dans lequel la lentille est une lentille plan-concave (402), ou
le dispositif émetteur optique implantable comprend en outre une virole (508, 805) positionnée à l'extrémité proximale du boîtier et configurée pour contenir les multiples fenêtres optiques (506a-506d), la virole présentant plusieurs ouvertures (510a-510d) alignées sur les multiples fenêtres optiques, les multiples fenêtres optiques étant en retrait par rapport à une surface supérieure de la virole, ou
les multiples sources d'émission de lumière (235) comprennent N sources d'émission de lumière et les multiples fenêtres optiques comprennent M fenêtres optiques, N étant supérieur ou égal à M.

5. Système selon la revendication 1, dans lequel l'élément optique comprend une seule fenêtre optique (502), éventuellement
dans lequel la fenêtre optique unique (502) comprend une lentille (402), un revêtement antireflet (404), une couche de diffusion (406), une surface microstructurée (408) ou toute combinaison de ceux-ci, ou
éventuellement, dans lequel le dispositif émetteur optique implantable comprend en outre une virole (508, 805) présentant plusieurs ouvertures (510a-510d), la virole étant positionnée à l'extrémité proximale du boîtier et la fenêtre optique unique (502) étant en retrait par rapport à l'extrémité proximale du boîtier d'au moins une épaisseur de la virole,
de préférence, dans lequel les multiples sources d'émission de lumière (235) comprennent N sources d'émission de lumière et les multiples ouvertures comprennent M ouvertures, N étant supérieur ou égal à M.

6. Système selon la revendication 1, dans lequel chacune des multiples sources d'émission de lumière (235) comprend un quelconque élément parmi une diode électroluminescente et un laser à cavité verticale émettant par la surface.

7. Système selon la revendication 1, dans lequel un premier groupe des multiples sources d'émission de lumière (235) comprend des lasers à cavité verticale émettant par la surface et un second groupe des multiples sources d'émission de lumière (235) comprend des diodes électroluminescentes.

8. Système selon la revendication 1, dans lequel les multiples sources d'émission de lumière (235) sont configurées pour fonctionner entre 400 nm et 1 400 nm, ou entre 600 nm et 1 300 nm.

9. Système selon la revendication 1, dans lequel les un ou plusieurs pilotes (230) sont configurés pour fonctionner selon une modulation tout ou rien, ou
dans lequel les un ou plusieurs pilotes (230) sont configurés pour fonctionner selon une modulation par déplacement d'amplitude multiple, ou
dans lequel le dispositif émetteur optique implantable comprend en outre une circuiterie frontale analogique configurée pour convertir un signal physiologique en un signal de données numériques.

10. Procédé de communication optique transcutanée, le procédé comprenant :
au niveau d'un dispositif émetteur optique implantable :
la conversion d'un signal de données numériques en un ou plusieurs signaux de modulation ;
au niveau d'une ou plusieurs de multiples sources d'émission de lumière (235), la génération d'un faisceau lumineux (130) en réponse à un signal de modulation correspondant des un ou plusieurs signaux de modulation, chaque faisceau lumineux (130) contribuant à former un signal optique combiné ; et
l'orientation du signal optique combiné afin qu'il sorte du dispositif émetteur optique implantable, distribué selon un modèle dans lequel une position de pic d'intensité de lumière de chaque faisceau lumineux du signal optique combiné est séparée d'une position de pic correspondante d'intensité de lumière d'un faisceau lumineux adjacent du signal optique combiné par au moins une première distance et moins d'une seconde distance, la première distance étant inférieure à la seconde distance, dans lequel le dispositif émetteur optique implantable est configuré pour être intégré dans un tissu biologique (207) et la première distance et la seconde distance sont basées sur des caractéristiques du tissu biologique (207).

11. Procédé selon la revendication 10, comprenant en outre :
au niveau d'un dispositif récepteur optique externe (110) positionné pour détecter un ou plusieurs des faisceaux lumineux (130) :
au niveau d'une ou plusieurs des multiples photodiodes (245), la détection de la lumière générée par les multiples sources d'émission de lumière (235) et la génération, en réponse, d'un signal de détection externe ;
l'amplification du signal de détection externe ; et
la réception du signal de détection amplifié et la génération d'un signal de données reconstruit.

12. Procédé selon la revendication 10, dans lequel la première distance est supérieure à 0,5 millimètre et la seconde distance est inférieure à 50 millimètres.

13. Procédé selon la revendication 10, dans lequel l'orientation est assurée par un élément optique comprenant plusieurs fenêtres optiques (506a-506d).

14. Procédé selon la revendication 10, dans lequel l'orientation est assurée par un élément optique comprenant une seule fenêtre optique (502).

15. Procédé selon la revendication 10, dans lequel chacune des multiples sources d'émission de lumière (235) comprend un quelconque élément parmi une diode électroluminescente et un laser à cavité verticale émettant par la surface, ou
dans lequel un premier groupe des multiples sources d'émission de lumière (235) comprend des lasers à cavité verticale émettant par la surface et un second groupe des multiples sources d'émission de lumière (235) comprend des diodes électroluminescentes, ou
dans lequel la génération d'un faisceau lumineux (130) en réponse à un signal de modulation correspondant des un ou plusieurs signaux de modulation est basée sur une modulation tout ou rien, ou
dans lequel la génération d'un faisceau lumineux (130) en réponse à un signal de modulation correspondant des un ou plusieurs signaux de modulation est basée sur une modulation par déplacement d'amplitude multiple, ou comprenant en outre la conversion d'un signal physiologique en signal de données numériques.
